(19) **Europäisches Patentamt**
**European Patent Office**
**Office européen des brevets**

(11) **EP 2 369 984 B1**

(12) **EUROPEAN PATENT SPECIFICATION**

(45) Date of publication and mention
of the grant of the patent:
**27.02.2019 Bulletin 2019/09**

(51) Int Cl.:
***A61B 5/022*** (2006.01)

(21) Application number: **09765156.6**

(86) International application number:
**PCT/GB2009/002772**

(22) Date of filing: **27.11.2009**

(87) International publication number:
**WO 2010/061197 (03.06.2010 Gazette 2010/22)**

(54) **METHOD OF MEASURING BLOOD PRESSURE AND APPARATUS FOR PERFORMING THE SAME**

VERFAHREN UND VORRICHTUNG ZUR BLUTDRUCKMESSUNG

PROCÉDÉ ET APPAREIL POUR DÉTERMINER LA PRESSION SANGUINEPROCÉDÉ ET APPAREIL POUR DÉTERMINER LA PRESSION SANGUINE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **28.11.2008 GB 0821781**

(43) Date of publication of application:
**05.10.2011 Bulletin 2011/40**

(73) Proprietor: **Royal United Hospital Bath NHS Trust Bath BA1 3NG (GB)**

(72) Inventors:
• **TOOLEY, Mark, Arthur**
 **Bath BA2 4RJ (GB)**
• **MAGEE, Patrick, Terence**
 **Bath BA2 7AT (GB)**

(74) Representative: **Akers, Noel James**
**N.J. Akers & Co**
**63 Lemon Street**
**Truro, TR1 2PN (GB)**

(56) References cited:
**WO-A1-99/13767**   **WO-A1-2007/017661**
**WO-A1-2007/069155**   **US-A- 5 830 131**

**Description**

The present invention relates to a method of measuring the blood pressure of a subject and to an apparatus for performing the same.

Measuring the blood pressure of a subject is a very common procedure in a great many medical procedures. The blood pressure of a subject may be measured using an invasive technique, in which a cannula is inserted into an artery of the subject. In many situations, the invasive techniques, while potentially more accurate for measuring and monitoring blood pressure, have associated risks and are not suitable or practical to be used. Accordingly, non-invasive techniques are very often preferred.

There are several techniques for the non-invasive measurement of blood pressure. In general, they function by occluding the flow of blood in the arteries of a limb, typically an arm, by inflating a proximal cuff applied to the limb. The cuff is gradually deflated and the onset of blood flow in the limb distal of the cuff is determined. The methods for detecting the onset of blood flow as the pressure in the cuff is reduced include palpation, auscultation, plethysmography, oscillotonometry and oscillometry.

Auscultation is one particularly commonly used technique and relies upon the medical practitioner or healthcare worker listening to the Korotkoff sounds in a region of the limb distal to the cuff as the pressure in the cuff is released. The Korotkoff sounds are described in terms of five phases as the pressure in the cuff is released and the flow of blood returns. With the cuff inflated to a pressure above systolic blood pressure (SBP), blood flow in the limb is prevented and no sounds are heard in the region of the limb distal to the cuff. As the pressure in cuff is reduced, Phase I of the Korotkoff sounds begin and a sharp tapping sound is heard as the cuff pressure reaches the SBP. Phase II sounds are heard as the cuff pressure is reduced and are characterised by softer murmers or swishing sounds. Further reduction of the cuff pressure leads to Phase III sounds, characterised by a resumption of harder tapping or thumping tones. Phase IV sounds is marked by an abrupt muffling of the Phase III sounds. Finally, further reduction in the pressure in the cuff leads to no sounds being audible, as the normal blood flow resumes through the blood vessel. Practice varies with respect to the recording of the diastolic blood pressure (DBP). Some practitioners take the onset of the Phase IV sounds as an indication of DBP. Others record the onset of Phase V as the DBP. The use of the Korotkoff sounds in measuring blood pressure is a difficult technique and requires training to be undergone by the medical practitioner or healthcare worker. This restricts the number of people able to measure a subject's blood pressure.

Auscultation is a simple and effective method for measuring blood pressure. However, it has been found that the method can be inaccurate, in particular in the determination of DBP. The method has been found to lead to overestimates of DBP at lower blood pressures and to result in underestimates of DBP at higher blood pressures. Other methods may be employed, which may provide more accurate measurements. However, they are generally more complicated to apply and, for this reason, are not generally favoured by many medical practitioners.

An accurate measurement of DBP is very useful in determining the general condition of a subject and in identifying certain conditions. For example, an accurate measurement of DBP is important in the diagnosis of hypertension. A high SBP is not necessarily an indicator of a medical condition. Indeed, healthy individuals are capable of mounting a high SBP, for example during stress and exercise, and a high SBP is not, by itself, necessarily an indication of an adverse medical condition. A high DBP, however, is of much greater concern and may be an indication of underlying arterial disease, showing a lack of compliance in the blood vessels. Conversely, as coronary artery perfusion occurs during diastole, a low DBP may contribute to inadequate coronary artery filling, possibly leading to heart failure.

Accordingly, it would be most advantageous if a simple and accurate non-invasive technique for measuring blood pressure could be provided. It would be particularly advantageous if the technique could provide an accurate indication of DBP. It would be further advantageous if the method could be employed by a wide range of people in the healthcare sector without needing to undergo extensive training.

Automated techniques for determining blood pressure using non-invasive techniques are known. In general, the methods are acceptable for determining SBP and the mean blood pressure (MBP). The SBP and MBP values are then used in an algorithm to estimate the DBP. A typical algorithm is as follows:

$$MBP = 1/3\ SBP + 2/3\ DBP.$$

It will be noted that the DBP is the most important component of blood pressure contributing to the MBP, according to the above equation.

Given the importance of the measurement of DBP as a diagnostic tool, there have been a number of attempts to provide improved techniques for its determination.

US 4,718,426 is concerned with a method for determining diastolic arterial blood pressure in a subject. The method comprises a first calibration phase, in which the blood pressure occurring in relation to various initial conditions of arterial blood is determined. From these measurements, the values of a plurality of coefficients are ascertained, each coefficient being associated with terms in a mathematical function characterising blood pressure values in relation to arterial wall displacement.

Thereafter, the method comprises undertaking a continuous monitoring phase, during which subsequently occurring arterial wall pressure displacement values are measured and blood pressure values are ascertained therefrom. These data are then used to recalibrate the system for measuring the blood pressure of the subject. The method and apparatus of US 4,718,426 is particularly complex and time consuming. A simpler and faster technique for accurately measuring DBP would be advantageous.

US 2002/0147402 is also concerned with the measurement of blood pressure, in particular DBP. US 2002/0147402 discloses a method including, in general terms: (a) generating first and second signals indicative of cardiac induced pulsatile variations of a cardiovascular parameter in first and second regions of the subject's body; (b) processing the first and second signals to derive values of a delay between pulses in the first signal and corresponding pulses in the second signal; (c) applying a variable pressure to a pressure application region of the subject's body so as to affect blood flow through at least one artery in the pressure application region, the variable pressure being varied over time, the first region, second region and the pressure application region being chosen such that the delay varies as a result of changes in the variable pressure; (d) deriving parameters of a mathematical function such that the function corresponds to a relationship between the delay and the variable pressure; (e) calculating a difference between the values of the delay and corresponding values in the mathematical function; and (f) identifying the DBP as a value of the variable pressure for which the difference exhibits a stationary point. Again, the method of US 2002/0147402 is particularly complex to operate and can be time consuming for the medical practitioner to complete fully.

GB 2,381,076 relates to a non-invasive method for determining DBP. The method employs a cuff inflated to occlude the blood flow through an artery in a limb of the subject. The cuff is deflated in a controlled manner. At a plurality of the deflation pressure levels, the pressure in the cuff is measured. In particular, oscillations in the cuff caused by the pressure pulses in the blood flow of the subject are measured. A waveform of the pressure oscillations within the cuff at various deflation pressures is generated and used to calculate the MAP and SBP. A portion of the waveform, generated at cuff pressures below DBP, is then calibrated by identify points corresponding to SBP, MBP and DBP. DBP is then derived as a function of those points and the earlier estimates of the MBP and SBP. It appears that the method of GB 2,381,076 again relies upon the derivation of DBP by calculation, rather than by direct measurement.

WO 2007/015153 is concerned with a non-invasive apparatus for estimating blood pressure. The apparatus measures a pulmonary component from detected heart sounds and analyses the pulmonic component to obtain a number of oscillations in the component. A predetermined relationship between the number of oscillations and blood pressure is then applied to estimate the blood pressure values. Again, this method relies upon an indirect estimate of blood pressure from other measured factors.

WO 2007/092680 discloses a method for measuring blood pressure, in particular SBP and DBP. The method employs a cuff that is inflated by a flow of fluid, typically air, therethrough. The pressure of the fluid flowing through the cuff is measured at both the systolic and diastolic points in the pressure cycle. The method corrects for the pressure losses in the flow of the fluid through the conduit connecting the pressure sensor to the cuff.

JP 2003135412 discloses a method and apparatus for measuring blood pressure. In order to reduce the discomfort to the patient, a fine vibrational pressure is applied to the occluding pressure at the cuff. In this way, the patient is subjected to less pressure.

WO 2007/017661 discloses a device for measuring blood pressure.

WO 2007/069155 discloses the Doppler detection of pulsatile blood flow in a patient.

Finally, WO 2008/015921 discloses a method and apparatus for measuring blood pressure in which a vibration is applied to the occluding pressure. The apparatus operates to ensure that the volume of the artery of the subject is kept constant and blood pressure is determined from the increased/decreased value of the applied pressure.

There is a need for an improved method of measuring the blood pressure of a subject, in particular a method that allows for an accurate measurement of DBP, that is both simple and quick to use by a medical practitioner. It would be most advantageous if the method could be made generally available to healthcare workers, without them having to undergo extensive training.

Accordingly, in a first aspect, the present invention provides a method for measuring the blood pressure of a subject, the method comprising:

applying an occluding pressure to a portion of the subject such that the flow of blood through a blood vessel may be at least partially occluded;
increasing the occluding pressure;
superimposing on the occluding pressure a pressure signature; and
monitoring the flow of blood through the blood vessel to identify a flow signature corresponding to the superimposed pressure signature.

In summary, in the method of the present invention, the flow of blood through a blood vessel, for example an artery in an arm of the subject has pressure applied to it. As the applied pressure is increased, the blood vessel is increasingly occluded, resulting in the flowrate of blood through the vessel being reduced and, when the pressure is high enough, being stopped. A pressure signature is applied together with the occluding pressure so as to be superimposed upon it. The pressure signature is a pressure wave having a predetermined and recognisable pattern, for example a series of pressure pulses. As discussed in more detail below, the pressure may comprise one or more, preferably two or more, positive pressure pulses, negative pressure pulses or combinations of the two. The pressure signature has the effect of modulating the occluding pressure to provide a pressure waveform bearing the characteristic pressure fluctuations of the signature. The flowrate of blood through the blood vessel is monitored downstream or distally of the point of application of the occluding pressure and pressure signature. The pressure signature is such that, at the appropriate occluding pressure, monitoring the flow of blood through the vessel can detect changes arising from the pressure signature being applied. In particular, it is preferred that the monitoring of the flow of blood distally is arranged to detect periods of zero blood flow through the blood vessel. The value of the occluding pressure that is being applied at this time is then used in a determination of the blood pressure. The pressure signature preferably has a complex waveform, more preferably with unequally spaced pressure pulses, in order to render the signature distinct and readily identifiable in the monitoring of the blood flow downstream.

In the first stages of the method, the occluding pressure and the superimposed pressure signature have a total pressure that is below the DBP of the subject. In this case, the blood pressure of the subject is such that the flowrate of blood through the blood vessel is substantially unaffected by the pressure being applied and the measured blood flowrate is of the normal flow pattern. As the occluding pressure is increased it approaches the DBP of the subject and the applied pressure begins to affect the flowrate of blood through the blood vessel. In particular, the pressure waveform characteristic of the applied pressure signature becomes recognisable as corresponding changes or modulations in the measured blood flowrate. Measurement of the occluding pressure being applied to the subject at the point that the characteristic signature is first detected in the monitored flowrate provides a value for the DBP of the subject. As noted above, it is particularly convenient if the pressure signature is such that periods of zero blood flow are generated in the blood vessel as the occluding pressure approaches the DBP of the subject.

The method may be terminated once the DBP has been measured. However, the method may be continued and the occluding pressure further increased. Once the applied occluding pressure approaches the SBP of the subject, the normal pattern of blood flowrate will disappear completely and the flow detector will be detecting a blood flowrate that follows only the pattern of the pressure signature. Measurement of the occluding pressure being applied to the subject at the point that the characteristic signature is the only waveform being measured provides a value for the SBP of the subject.

If desired, the MBP of the subject may be calculated from the measured DBP and SBP, for example using the algorithm set out above.

The method of the present invention comprises applying an occluding pressure to a portion of the subject. In this respect, the term 'occluding pressure' is a reference to the application of pressure to the subject in a manner that, providing the pressure is high enough, can occlude a blood vessel in the portion of the subject such that the flow of blood through the blood vessel is stopped, either in the whole waveform or, depending upon the pressure applied, certain portions of the waveform. The occluding pressure may be applied to any suitable portion of the subject, that allows a significant blood vessel to be occluded. The suitable portion is preferably a limb, in particular an arm of the subject, in keeping with common practices in the art. The occluding pressure may be applied by any suitable means, most preferably a cuff, as described in more detail hereinafter. The occluding pressure is applied to bear upon a blood vessel within the subject, such that increasing the occluding pressure will restrict and eventually stop the flow of blood through the vessel, as the occluding pressure is increased. The blood vessel targeted in this way may be any suitable vessel and is most preferably an artery, with the method determining the systolic arterial pressure (SAP) and/or the diastolic arterial pressure (DAP) of the subject.

In the method, the occluding pressure is increased. If the DBP of the subject is to be measured, the occluding pressure is increased from a value below the DBP, with the starting pressure being such that the total pressure of the occluding pressure and the pressure signature is below the DBP. Thereafter, the occluding pressure is increased. The occluding pressure may be increased in any suitable pattern, for example continuously or stepwise. Most preferably, the occluding pressure is increased continuously. The rate of increase of the occluding pressure may be any suitable rate, provided that the accurate determination of the DBP is possible.

The occluding pressure may be increased at any suitable rate. The rate of increase of the occluding pressure should be slow enough that the blood pressure, in particular the DBP and SBP, can be measured accurately. In one preferred embodiment, the occluding pressure is increased stepwise or continuously in stages, the increase in each stage corresponding to the amplitude of the pressure signature waveform. The occluding pressure may be increased at any suitable stage in or continuously throughout the blood pressure cycle of the subject. In one preferred embodiment, the increases in the occluding pressure are related or synchronised with the frequency of the blood pressure cycle of the subject. In particular, the occluding pressure is increased during the systolic portion of each pressure cycle. In this way, the method may be adapted to the blood pressure cycle of the subject, as the pattern of the blood pressure cycle may vary significantly from subject to subject. This, in turn, allows the method to provide an optimum determination of blood pressure for each subject.

In addition to the occluding pressure, the method requires the application of a pressure signature to the subject. The pressure signature is a pressure signal applied together with, that is superimposed on, the occluding pressure, that is applied to modify or modulate the waveform of the occluding pressure. The pressure signature applied to the occluding pressure is significantly smaller in value than the occluding pressure itself. In particular, the amplitude of the pressure signature waveform should be significantly lower than the value of the occluding pressure applied throughout the method. Preferably, the amplitude of the pressure signature waveform is less than 10% of the value of the occluding pressure being applied, more preferably less than 5%, still more preferably less than 3%. In this way, the detection of the onset of variations in the blood flow in the vessel as a result of the applied pressure signature will occur at an occluding pressure that is substantially the same as the DBP of the subject.

As noted, in order to provide the greatest accuracy of the determination of the blood pressure, in particular the DBP, it is preferred that the amplitude of the pressure signature waveform is low, relative to the occluding pressure. This is particularly the case where the pressure signature comprises one or more, more preferably two or more, positive pressure pulses. If required, for greater accuracy, the value of DBP and SBP provided by the method may be corrected to take account of the pressure signature. In particular, in the case of a pressure signature comprising one or more positive pressure pulses, the value of DBP may be increased by the value of the or each pressure pulse, to provide a more accurate reading of the actual DBP of the subject.

The pressure signature must be of such a form that the waveform of total pressure actually applied to the subject affects the flow of blood through the blood vessel being occluded. In this way, a pattern of changes in the flow of blood corresponding to the features of the pressure signature can be detected. The pressure signature may have any suitable waveform that affects the flow of blood through the blood vessel and can be measured distally. The pressure signature may have a waveform comprising continuous changes in pressure and/or intermittent changes or pulses of pressure. In one preferred embodiment, the pressure signature is characterised by one or more pressure pulses applied to the occluding pressure waveform. Single pressure pulses may be applied. Alternatively, the pressure pulses may be applied in groups of two or more pulses, so as to provide a more recognisable signature. The amplitude and frequency of the pressure signature waveform should be sufficient to affect the flowrate of blood through the blood vessel such that the changes in the flowrate can be measured, as noted above. In addition, the amplitude and frequency of the pressure signature waveform should be readily distinguishable from the general or background noise that will exist in the system of measuring the flowrate of blood through the blood vessel.

Suitable pressure signature waveforms will be readily designed by the person skilled in the art. It is preferred that the frequency or time period of the pressure signature waveform is no shorter than the time period of the diastolic portion of the blood pressure cycle of the subject. In this way, it is ensured that any changes in the blood flow resulting from the applied pressure signature will be detectable during the diastolic portion of every blood pressure cycle in the blood vessel. More preferably, the frequency or time period of the pressure signature is preferably at least twice, even more preferably at least three times the length of the diastolic period of the blood pressure cycle, such that the pressure signature may be detected at least twice, more preferably at least three times, during the diastolic period of each cycle.

The method of the present invention further comprises measuring the flow of blood through the blood vessel being occluded. The flow of blood in the vessel is measured downstream or distally of the point of application of the occluding pressure. The flow of blood may be measured using any suitable technique and methods for measuring the flow of blood through a blood vessel within a subject are known in the art. Non-invasive flow measuring techniques are especially preferred. In particular, when applying the method to measure the DBP, the method is identifying when the flow of blood through the blood vessel drops to zero during the diastolic phase of the blood pressure cycle, following the pattern of the applied pressure signature. Accordingly, the method may employ any technique that is particularly suitable for detecting when the blood flow intermittently falls to zero. One preferred method of measuring the flow of blood through the blood vessel is using a device that relies upon the Doppler effect. Again, suitable Doppler devices for measuring the blood flow are known in the art and are commercially available.

In one preferred embodiment, the method employs a flow detector comprising a plurality of Doppler sensors disposed at varying positions on the subject. The method includes selecting from the array of Doppler sensors the sensor that provides the optimum or most accurate monitoring of the blood flow, so as to detect changes in the blood flow resulting from the applied pressure signal. This may be determined, for example, by analysing the waveform output by the sensors in the array representing the flow of blood throughout several cycles of the subject's heart and selecting the sensor providing the best flow signal, in particular the sensor or sensors providing the cleanest and clearest signal with the highest power.

Alternatively, the method may be carried out using the signals output from more than one Doppler sensor in the array, for example by generating a combined trace of the blood flow by averaging the signal outputs.

The Doppler sensor is not required to quantify the signature waveform being detected. Rather, it is merely necessary for the blood flow sensor to detect the signature and the presence of zero blood flow in the blood vessel.

As noted above, the flow of blood through the blood vessel is measured in order to detect changes in the flow pattern that correspond to the characteristics of the pressure signature applied to modulate the occluding pressure. The method may include varying the pressure signature applied, for example by varying the amplitude and/or frequency of the pressure signature waveform, in order to improve the response of the flow pattern to the changes in applied pressure. For example, the conditions of one subject's arteries may require a pressure signature having a higher amplitude, in order to register a corresponding change in the flow pattern of blood through the blood vessel being monitored and have the method function efficiently. The method may therefore include a self-calibration routine, that adapts the parameters of the method, such as the form of the pressure signature waveform, to the particular condition of the subject.

It has been found that the method of the present invention allows for the blood pressure of a subject to be measured directly, in particular for the DBP and/or the SBP to be measured directly, without the need for estimating either one or both values from other measured parameters. In addition, the method is particularly quick to apply.

The method of the present invention has been found to be particularly well suited to measuring the blood pressure, in particular DBP and SBP, of a wide range of subjects with a diverse range of conditions. However, it may be that the method is not able to provide a blood pressure measurement for some subjects. The method relies upon the application of the occluding pressure to bear upon a blood vessel and vary, in particular reduce, the flow of blood therethrough. In some subjects, for example those with severe hardening of the arteries or other blood vessels, it may not be possible to apply an occluding pressure to vary the blood flowrate, as required to properly perform the method. In particular, it may not be possible to identify changes in the pattern of blood flow in the blood vessel arising from the pressure signature being applied. In such a case, the method will not provide a measurement of the blood pressure. In such cases, the method may be adapted such that, in the event that a blood pressure value cannot be determined, the method provides an indication to the healthcare worker of this failure. This will indicate to the user that the subject may be suffering from a serious condition, such as a severe hardening of the arteries.

In a further aspect, the present invention provides an apparatus for measuring blood pressure, as defined in the claims.

The apparatus of the present invention comprises means for applying pressure to a portion of the subject, whereby the flow of blood through a blood vessel in the subject may be restricted and occluded, as the pressure is increased. Any suitable means for applying pressure to the subject may be employed. A most preferred means is a cuff comprising a bladder for extending around a limb of the subject, in particular the arm. When the cuff is in position around the upper arm of a subject, inflation of the bladder with a suitable fluid, most especially air, will bring pressure to bear on a blood vessel within the arm, in particular a major blood vessel, such as an artery, especially the brachial artery. When the bladder is inflated to a sufficiently high pressure, the pressure on the blood vessel is sufficient to prevent the flow of blood therethrough.

In use, the pressure applied to the portion of the subject is increased. Accordingly, the apparatus comprises a means for increasing the applied occluding pressure. In a preferred embodiment, the means is a pump for supplying fluid to the bladder of the cuff, such that the bladder is slowly inflated, thereby increasing the pressure applied to the portion of the subject. The means may be manually operated. More preferably, the means is automated, in particular with the rate of increase of the occluding pressure being variable by the user or operator.

As noted above, the increase in the occluding pressure may be synchronised with the frequency of the blood pressure cycle of the subject. Accordingly, the apparatus of the present invention may also comprise means for monitoring the blood pressure cycle of the subject. Suitable means are known in the art. Alternatively, the means for applying the occluding pressure may be linked to the means for monitoring the flow of blood through the blood vessel and react to the blood flow cycle, which in turn is itself determined by the blood pressure cycle of the subject.

In addition, the apparatus comprises means for applying a pressure signature to the occluding pressure. The means may be a separate means for applying pressure in the signature waveform, for example a second pump for supplying pressurised fluid in a signature pattern to the bladder of the cuff or a means for compressing the bladder in the cuff to increase the fluid pressure therein according to the pressure signature. Alternatively, the means for applying the pressure signature may be same means as for applying the occluding pressure. For example, a pump for supplying fluid to the bladder of a cuff may be used to apply the occluding pressure, with the pressure increasing at the required rate, and for providing a pattern of pressure pulses or waves to the fluid stream according to the pressure signature.

Further, the apparatus comprises means for monitoring the flow of blood through the blood vessel. In particular, means are provided for measuring the flow of blood downstream or distal of the point of application of the occluding pressure. Any suitable means for measuring blood flow through the vessel may be employed. Non-invasive means are most especially preferred. Suitable means are known in the art. In particular, the means are preferably able to detect the lack of blood flow or zero flow within the blood vessel, especially intermittent zero flow. One preferred means for measuring the flow of blood in a non-invasive manner is a Doppler sensor. Doppler sensors and their use in measuring blood flow within a vessel are known in the art and suitable sensors are commercially available. In a preferred embodiment, the apparatus comprises an array of blood flow sensors, in particular Doppler sensors. The sensors in the array are arranged in different orientations with respect to the target blood vessel. In this way, the best positioned sensor may be selected from the array, in order to provide the indication of blood flow through the vessel. In this case, the apparatus further comprises means for selecting a sensor from an array comprising a plurality of sensors.

The sensor for measuring the blood flow through the vessel, or the array of such sensors, may be disposed at any suitable location relative to the means for applying the occluding pressure, such that the blood flow downstream or distal of the point of application of the occluding pressure is measured. In one preferred arrangement, the apparatus comprises two cuffs, the first cuff as hereinbefore described having an inflatable bladder, the second cuff for extending around a limb of the subject and housing the one or more flow sensors. The two cuffs may be mountable separately on the subject, for example around one limb of the subject. Alternatively, the apparatus may comprise a single cuff having a first and second cuff portions, as just described.

The apparatus further comprises a means for identifying a flow pattern of blood through the blood vessel that corresponds to a characteristic pattern of the pressure signature being applied. The sensor detecting the flow of blood through the blood vessel of the subject will output a signal. The means for identifying the flow pattern may be any suitable processor that can receive and process the signal produced by the flow sensor.

The apparatus may further comprise other components as may be required, for example a suitable display to provide an indication of the determined blood pressure values, in particular the DBP and/or SBP, as required. The apparatus may be provided with a control means, whereby a user may select the mode of operation of the system, for example to select the measurement of DBP only, the measurement of SBP only or the measurement of both DBP and SBP. The apparatus may then be operated according to the selection of the user, in particular to apply the occluding pressure appropriate to the determination selected. A suitable processor may be provided to control the components of the apparatus.

The apparatus of the present invention may be provided in a compact unit that is easy to apply to the subject and operate, with little or no discomfort to the subject. In particular, the apparatus may be provided, in one embodiment, with a cuff assembly having a bladder and housing one or more pumps to provide the occluding pressure and apply a pressure signature to the bladder, an array of one or more means for monitoring the flow of blood through the blood vessel, as well as one or more processors to control the operation of the cuff assembly and process the signals received from the flow sensors. This self-contained cuff assembly may be connected to a remote display and/or control panel, for example by a wireless connection.

As noted above, the apparatus of the present invention, in one preferred embodiment, employs an array of flow sensors disposed on the subject, with the flow sensors being at different positions. This allows the sensor in the optimum position for monitoring the blood flow in the blood vessel to be identified and used in any measurement. Such an array of flow sensors may be used in a wide range of systems for measuring blood pressure and monitoring blood flow and is not limited to the apparatus of the present invention.

The present invention will now be described, by way of example only, having reference to the accompanying drawings, in which:

Figure 1 is a typical trace of the blood pressure of a subject plotted against time;

Figure 2 is the trace of Figure 1 showing traces of the applied occluding pressure and pressure signature of a first stage of the method of the present invention;

Figure 3 is the trace of Figure 2 showing the pressure traces in a second stage of the method of the present invention for a determination of the DBP of the subject;

Figure 4 is the trace of Figure 2 showing the pressure traces in a third stage of the method of the present invention for a determination of the SBP of the subject; and

Figure 5 is a diagrammatic representation of an apparatus according to the present invention.

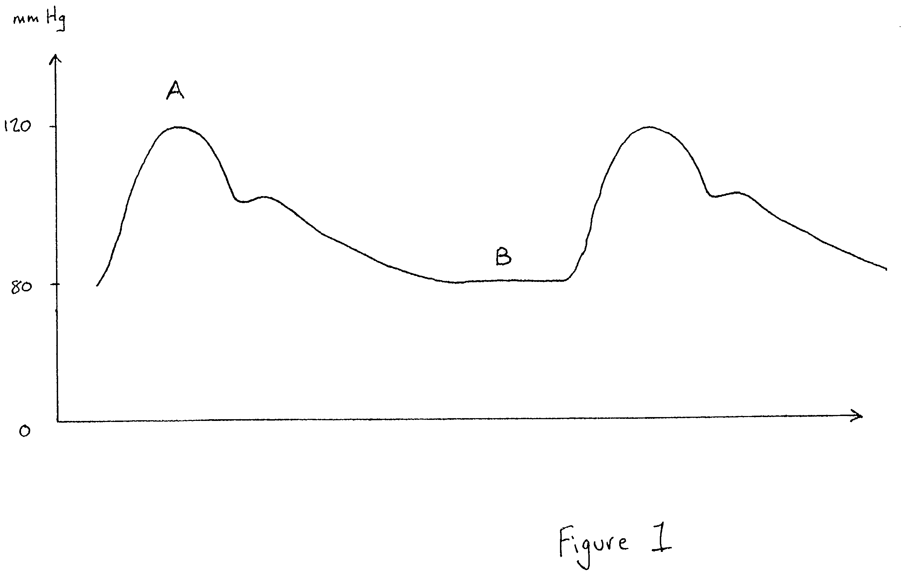

Referring to Figure 1, there is shown a typical trace of blood pressure of a subject plotted against time, to show the variations in pressure within a blood vessel throughout the blood pressure cycle. The blood pressure trace has a systolic portion A, indicating a SBP of about 120 mm Hg, and a diastolic portion B, indicating a DBP of about 80 mm Hg. The flow of blood through a blood vessel, for example an artery such as the brachial artery in the arm, of the subject, will follow a similar trace to that of Figure 1, with the blood flowrate being higher at higher pressures and lower at lower pressures.

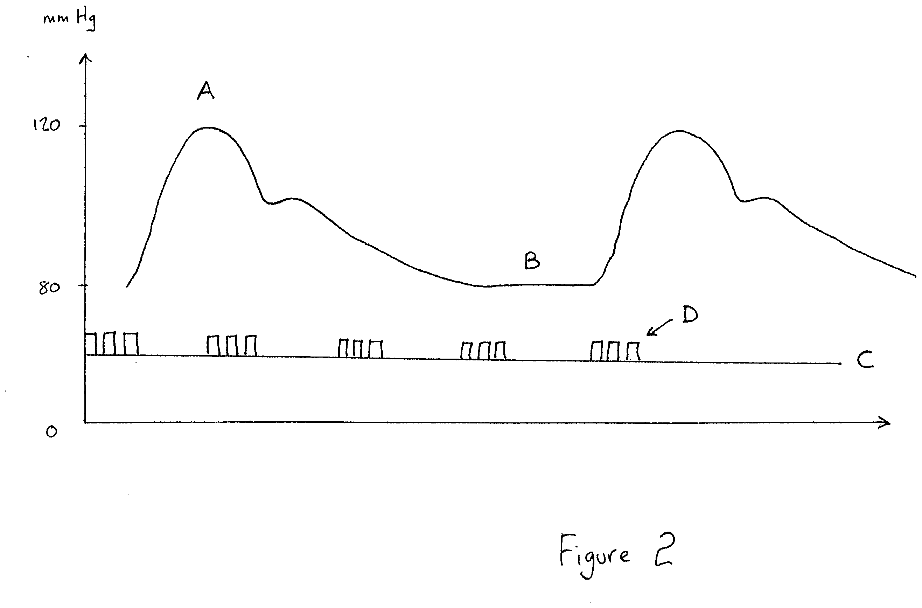

Referring to Figure 2, there is shown the blood pressure trace of Figure 1, together with the trace of pressure applied to a portion of the subject's body by the method of the present invention. The pressure is applied by the method, for example using a cuff having an inflatable bladder extending around an arm of the subject and applying pressure to the brachial artery of the subject, thereby affecting the flow of blood through the artery. The pressure applied in the method comprises an occluding pressure, indicated in Figure 2 by line C. The occluding pressure C in Figure 2 is shown as being a substantially constant pressure of below 80 mm Hg, as indicated by the waveform in the form of a horizontal line. The occluding pressure waveform is modified by an applied pressure signature, indicated as D in Figure 2. The pressure signature has a pulsed waveform, comprising groups of pressure pulses, each group having three pressure pulses. It will be appreciated that the waveform of the pressure signature shown in Figure 2 is just one form that may be applied in the present invention. The peak pressure of the occluding pressure waveform as modified by the pressure signature pulses is below the DBP of the subject. Accordingly, the blood pressure of the subject throughout the pressure cycle is sufficient to overcome the pressure applied by the method and no variation in the blood flow through the blood vessel occurs.

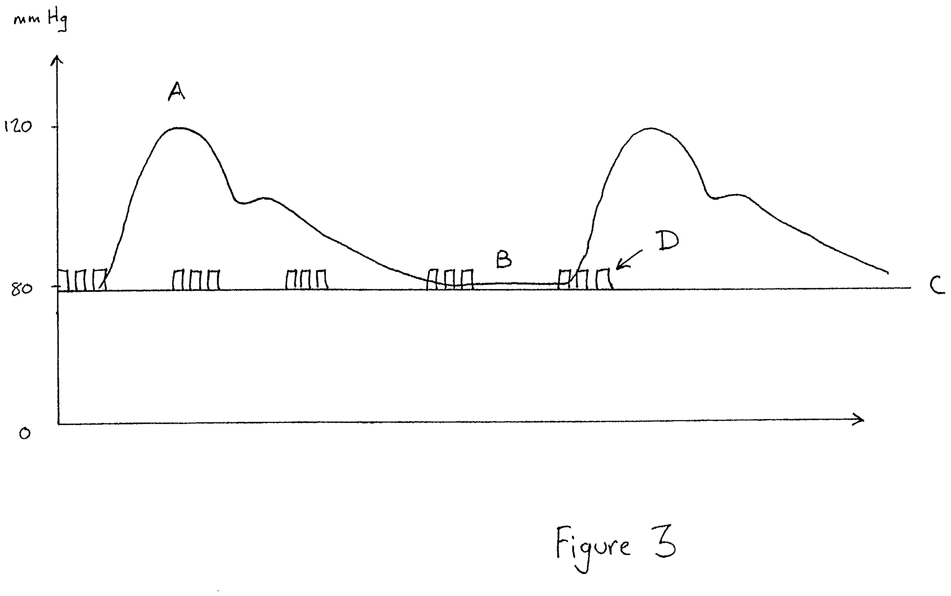

The occluding pressure applied in the method is slowly increased, such that it approaches the DBP of about 80 mm Hg. This situation is shown in Figure 3. As can be seen, during the diastolic phase of the blood pressure cycle, the peak pressure of the pressure pulses of the pressure signature exceeds the DBP. The affect on the flow of blood through the blood vessel of the subject is to impart to the flow pattern pulses corresponding to the pressure pulses of the pressure signature. As the occluding pressure is increased, these pulses in the flow pattern can be detected by measuring the flow of blood through the blood vessel distal of the point at which the occluding pressure is being applied. In particular, the blood flow will fall to zero during the diastolic phase B, as a result of the total pressure of the applied pressure pulses exceeding the DBP of the subject and closing the artery. This is characterised by pulses of zero flow occurring during the diastolic phase B. By identifying the first onset of these pulses of zero flow in the flow pattern corresponding to the pulses in the pressure signal being applied, the DBP can be determined to be the value of the occluding pressure being applied at that instant. The smaller the amplitude of the pressure signature in relation to the occluding pressure being applied, the higher the accuracy of the determination of DBP.

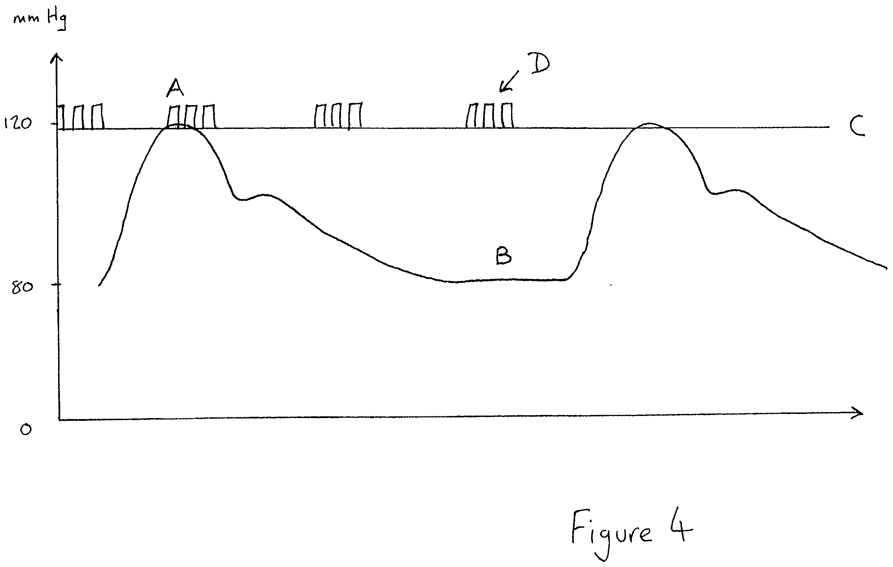

The method of the present invention may be ended once the DBP has been determined, if required. However, further increasing the occluding pressure being applied allows the SBP to be determined. Referring to Figure 4, there is represented the situation that the occluding pressure has been increased to approach the SBP of the subject. As the occluding pressure is increased, the variations in blood flow through the blood vessel resulting from the normal blood pressure cycle are increasingly masked by the pressure being applied to the subject. As the occluding pressure reaches the SBP of the subject, the blood flow through the blood vessel will be zero throughout the majority of the pressure cycle, with periods of blood flow occurring only during the systolic phase A. At the instant the pulses of blood flow during the systolic phase A correspond to the pressure pulses of the pressure signature being applied, the occluding pressure is substantially the same as the SBP and the SBP may be determined. Again, the smaller the amplitude of the pressure signature in relation to the occluding pressure being applied, the higher the accuracy of the determination of SBP.

The method of the present invention is ended at this point, as further increases in the occluding pressure merely closes the blood vessel completely and prevent all flow of blood.

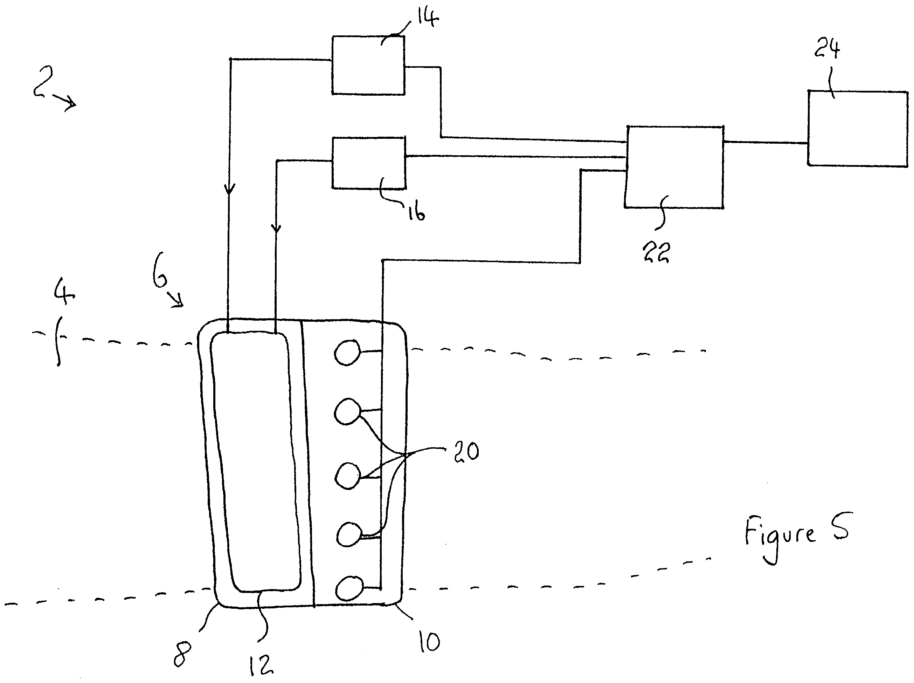

Referring to Figure 5, there is shown a diagrammatic representation of an exemplary apparatus. The apparatus, generally indicated as 2, is shown applied to the arm 4 of a subject, shown by a dashed line.

The apparatus comprises a cuff assembly 6 having a first cuff portion 8 and a second cuff portion 10. The cuff assembly 6 is applied around the arm of the subject such that the second cuff portion 10 is distal of the first cuff portion 8. The cuff assembly 6 applies pressure to the brachial artery of the arm of the subject. The two cuff portions 8, 10 are shown as being together in Figure 5. However, the two cuff portions may be spaced apart along the arm 4, as required by the user.

The first cuff portion comprises an inflatable bladder 12, that may be inflated by the supply of a pressurised fluid, in particular air. A pump 14 provides air under pressure to the bladder 12, causing the bladder to inflate. The inflation of the bladder 12 under the action of the air supplied by the pump 14 applies the occluding pressure of the method of the present invention to the arm of the subject. A second pump 16 provides a second supply of pressurised air to the bladder 12. The second pump 16 is operated to provide the air at a varying pressure, so as to provide a pressure signature of a predetermined waveform to the bladder. The combined air pressure waveforms delivered by the pumps 14 and 16 is applied as pressure to the arm of the subject by the bladder 12. In an alternative arrangement, the pump 14 may provide both the occluding pressure and be operated to provide the pressure signature, in which case the second pump 16 may be omitted from the system.

The second cuff portion 10 housing an array of Doppler sensors 20 disposed around the arm of the subject. The Doppler sensors are responsive to the flow of blood through the brachial artery. In particular, the sensors 20 indicate zero flow of blood through the artery. The output signals from the Doppler sensors 20 are transmitted to a processor 22. The processor 22 is also arranged to monitor the occluding pressure being applied to the arm by the bladder 12, for example by monitoring the pressure within the bladder and/or by controlling the pump 14. The processor 22 is further arranged to control the amplitude and form of the pressure signature waveform being generated by the pump 16. This may be done in response to signals received from the sensors 20 relating to the blood flow. In this respect, the processor may derive the form, in particular the frequency, of the blood pressure cycle of the subject and adjust the pressure signature waveform accordingly. In addition, the processor may control the increase in the occluding pressure by means of the pump 14 in a similar manner. Alternatively, the apparatus 2 may comprise a separate monitor for measuring the blood pressure cycle of the subject (not shown for clarity) to provide the necessary data to the processor 22.

As noted, the second cuff portion 10 comprises an array of Doppler sensors 20. Depending upon the location and orientation of the second cuff portion with respect to the arm of the subject, some of the sensors may be in a better position to respond to changes in the blood flow through the artery in the arm than others. The processer 22 is adapted to select the output signals from one or more of the sensors, in order to optimise the determination of the blood pressure levels.

Finally, the apparatus 2 comprises a display 24 for providing such information as the details of the operating parameters of the system and the determined blood pressure, in particular the DBP and SBP.

The apparatus 2 has been shown in Figure 5 as comprising a number of separate components. However, the apparatus may be assembled in a compact format, with one or both of the pumps 14, 16 and/or the processor housed within the cuff assembly 6. Further, the link between the processor and one or more of the other components, although shown as a wire connection in Figure 5, may be wireless. In particular, the display 24 may receive signals and data from the processor 22 wirelessly.

The method and apparatus of the present invention allow the blood pressure of a subject, in particular the DBP and, if required, the SBP, to be measured in a simple and non-invasive manner, without significant intervention on the part of the healthcare worker. In particular, extensive training, such as is required in order to employ the Korotkoff sounds, is not required.

**Claims**

1. A method for measuring the blood pressure of a subject, the method comprising:

   applying an occluding pressure to a portion (4) of the subject such that the flow of blood through a blood vessel may be at least partially occluded; and
   increasing the occluding pressure;
   **characterised in that** the method further comprises:

   superimposing on the occluding pressure a pressure signature;
   monitoring the flow of blood through the blood vessel to identify a flow signature corresponding to the superimposed pressure signature; and
   to determine the blood pressure based on the identified flow signature.

2. The method according to claim 1, wherein the occluding pressure is applied to a limb (4) of the subject, preferably wherein the limb is an arm (4), the occluding pressure bearing upon the brachial artery in the arm (4) of the subject.

3. The method according to any preceding claim, wherein the occluding pressure is increased in a stepwise or a continuous pattern.

4. The method according to any preceding claim, wherein the occluding pressure is increased from an initial value below the DBP of the subject, the method determining the DBP of the subject and/or wherein the occluding pressure is increased to a value approaching the SBP, the method determining the SBP of the subject.

5. The method according to any preceding claim, wherein the increase in the occluding pressure is synchronised with the blood pressure cycle of the subject.

6. The method according to any preceding claim, wherein the pressure signature has a waveform having an amplitude less than 10% of the value of the occluding pressure, more preferably less than 5%.

7. The method according to any preceding claim, wherein the pressure signature has a waveform comprising a plurality of pressure pulses, preferably wherein the waveform comprises a plurality of groups of pressure pulses, each group comprising a plurality of individual pressure pulses.

8. The method according to any preceding claim, wherein a plurality of flow monitoring sensors are disposed on the subject, preferably wherein the method further comprises selecting one or more sensors from the plurality of sensors to optimise the blood pressure measurement.

9. The method according to any preceding claim, wherein, in the event a measurement of the blood pressure of the subject cannot be provided, an indication regarding the same is provided to the user.

10. An apparatus (2) for measuring blood pressure, the apparatus comprising:

    means (6) for applying an occluding pressure to a portion (4) of a subject, such that the flow of blood through a blood vessel may be occluded;
    means (14) for increasing the occluding pressure; and
    a sensor (20) for detecting changes in the flow of blood through the blood vessel as a result of the applied pressure;

**characterised by** further comprising:

means (16) for applying a pressure signature superimposed on the occluding pressure to the portion (4) of the subject; means (22) for determining when the changes in the flow of blood correspond to the characteristics of the pressure signature; and means to determine the blood pressure on the basis of the changes in the flow of blood.

11. The apparatus (2) according to claim 10, wherein the means (6) for applying the occluding pressure to the subject comprises a cuff for extending around a limb (4) of the subject, preferably wherein the cuff comprises a first cuff portion (8) for applying the occluding pressure to the subject and a second cuff portion (10) having one or more blood flow sensors (20) therein.

12. The (2) apparatus according to either of claims 10 or 11, wherein the means (14) for applying the occluded pressure and the means (16) for applying the pressure signature are the same.

13. The apparatus (2) according to any of claims 10 to 12, wherein the sensor (20) for detecting changes in the flow of blood through the blood vessel is responsive to zero blood flow.

14. The apparatus (2) according to any of claims 10 to 13, wherein the sensor is a Doppler sensor (20).

15. The apparatus according to any of claims 10 to 14, comprising a plurality of sensors disposed in an array (20), so as to be disposed around the blood vessel of the subject when the apparatus (2) is in use, preferably further comprising means for selecting the output from one or more of the plurality of sensors in the array (20).

**Patentansprüche**

1. Verfahren zum Messen des Blutdrucks einer Versuchsperson, wobei das Verfahren Folgendes umfasst:

Anwenden eines Okklusionsdrucks auf einen Teil (4) der Versuchsperson, so dass der Blutfluss durch ein Blutgefäß wenigstens teilweise verschlossen werden kann; und Erhöhen des Okklusionsdrucks; **dadurch gekennzeichnet, dass** das Verfahren ferner Folgendes umfasst:

Überlagern des Okklusionsdrucks mit einer Drucksignatur; Überwachen des Blutflusses durch das Blutgefäß, um eine Strömungssignatur zu identifizieren, die der überlagerten Drucksignatur entspricht; und Bestimmen des Blutdrucks basierend auf der identifizierten Strömungssignatur.

2. Verfahren nach Anspruch 1, wobei der Okklusionsdruck auf ein Glied (4) der Versuchsperson ausgeübt wird, wobei das Glied vorzugsweise ein Arm (4) ist, wobei der Okklusionsdruck auf die Arteria brachialis im Arm (4) der Versuchsperson ausgeübt wird.

3. Verfahren nach einem der vorhergehenden Ansprüche, wobei der Okklusionsdruck nach einem schrittweisen oder kontinuierlichen Muster erhöht wird.

4. Verfahren nach einem der vorhergehenden Ansprüche, wobei der Okklusionsdruck von einem Anfangswert unter dem DBD der Testperson erhöht wird, wobei das Verfahren den DBD der Testperson bestimmt und/oder wobei der Okklusionsdruck auf einen Wert erhöht wird, der sich dem SBD nähert, wobei das Verfahren den SBD der Testperson bestimmt.

5. Verfahren nach einem der vorhergehenden Ansprüche, wobei der Anstieg des Okklusionsdrucks mit dem Blutdruckzyklus der Testperson synchronisiert wird.

6. Verfahren nach einem der vorhergehenden Ansprüche, wobei die Drucksignatur eine Wellenform mit einer Amplitude von weniger als 10 % des Wertes des Okklusionsdrucks aufweist, besonders bevorzugt weniger als 5 %.

7. Verfahren nach einem der vorhergehenden Ansprüche, wobei die Drucksignatur eine Wellenform aufweist, die eine Vielzahl von Druckpulsen umfasst, wobei die Wellenform vorzugsweise eine Vielzahl von Gruppen von Druckpulsen umfasst, wobei jede Gruppe eine Vielzahl von einzelnen Druckpulsen umfasst.

8. Verfahren nach einem der vorhergehenden Ansprüche, wobei eine Vielzahl von Strömungsüberwachungssensoren an der Testperson angeordnet sind, wobei das Verfahren ferner vorzugsweise das Auswählen eines oder mehrerer Sensoren aus der Vielzahl von Sensoren umfasst, um die Blutdruckmessung zu optimieren.

9. Verfahren nach einem der vorhergehenden Ansprüche, wobei in dem Fall, wenn eine Messung des Blutdrucks des Subjekts nicht bereitgestellt werden kann, dem Benutzer diesbezüglich ein Hinweis gegeben wird.

10. Vorrichtung (2) zum Messen des Blutdrucks, wobei die Vorrichtung umfasst:

Mittel (6) zum Anlegen eines Okklusionsdrucks auf einen Teil (4) einer Testperson, so dass der Blutfluss durch ein Blutgefäß verschlossen werden kann;
Mittel (14) zum Erhöhen des Okklusionsdrucks; und
einen Sensor (20) zum Erfassen von Änderungen im Blutfluss durch das Blutgefäß als Ergebnis des ausgeübten Drucks;
**dadurch gekennzeichnet, dass** es ferner Folgendes umfasst:

Mittel (16) zum Anwenden einer Drucksignatur, die auf den Okklusionsdruck auf den Teil (4) der Testperson angewendet wird;
Mittel (22) zum Bestimmen, wann die Änderungen des Blutflusses den Eigenschaften der Drucksignatur entsprechen; und
Mittel zum Bestimmen des Blutdrucks basierend auf den Änderungen im Blutfluss.

11. Vorrichtung (2) nach Anspruch 10, wobei das Mittel (6) zum Anwenden des Okklusionsdrucks auf die Testperson eine Manschette umfasst, die sich um ein Glied (4) der Testperson erstreckt, wobei die Manschette vorzugsweise einen ersten Manschettenteil (8) zum Anwenden des Okklusionsdrucks auf die Testperson und einen zweiten Manschettenteil (10) mit einem oder mehreren Blutflusssensoren (20) darin umfasst.

12. Vorrichtung (2) nach einem der Ansprüche 10 oder 11, wobei das Mittel (14) zum Anwenden des Okklusionsdrucks und das Mittel (16) zum Anwenden der Drucksignatur gleich sind.

13. Vorrichtung (2) nach einem der Ansprüche 10 bis 12, wobei der Sensor (20) zum Erfassen von Änderungen im Blutfluss durch das Blutgefäß auf einen Blutfluss von Null anspricht.

14. Vorrichtung (2) nach einem der Ansprüche 10 bis 13, wobei der Sensor ein Doppler-Sensor (20) ist.

15. Vorrichtung nach einem der Ansprüche 10 bis 14, die eine Vielzahl von Sensoren umfasst, die in einer Anordnung (20) angeordnet sind, um um das Blutgefäß der Testperson herum angeordnet zu werden, wenn die Vorrichtung (2) verwendet wird, vorzugsweise ferner Mitteln zum Auswählen der Ausgabe von einem oder mehreren der Vielzahl von Sensoren in der Anordnung (20) umfassend.

**Revendications**

1. Procédé de mesure de la pression artérielle d'un sujet, le procédé comprenant :

l'application d'une pression d'occlusion à une partie du corps (4) du sujet de façon à ce que l'écoulement du sang à travers un vaisseau sanguin puisse être au moins partiellement bloqué ; et
l'augmentation de la pression d'occlusion ;
**caractérisé en ce que** le procédé comprend en outre :

la superposition sur la pression d'occlusion d'une signature de pression ;
le suivi de l'écoulement du sang à travers le vaisseau sanguin pour identifier une signature d'écoulement correspondant à la signature de pression superposée ; et
la détermination de la pression artérielle sur la base de la signature d'écoulement identifiée.

2. Procédé selon la revendication 1, dans lequel la pression d'occlusion est appliquée à un membre (4) du sujet, de préférence dans lequel le membre est un bras (4), la pression d'occlusion se portant sur l'artère brachiale du bras (4) du sujet.

3. Procédé selon l'une quelconque des revendications précédentes, dans lequel la pression d'occlusion est augmentée selon un schéma par étape ou continu.

4. Procédé selon l'une quelconque des revendications précédentes, dans lequel la pression d'occlusion est augmentée à partir d'une valeur initiale se situant au-dessous de la pression artérielle diastolique (TAD) du sujet, le procédé déterminant la TAD du sujet et/ou dans lequel la pression d'occlusion est augmentée jusqu'à une valeur approchant la TAD, le procédé déterminant la TAD du sujet.

5. Procédé selon l'une quelconque des revendications précédentes, dans lequel l'augmentation de la pression d'occlusion est synchronisée avec le cycle de pression artérielle du sujet.

6. Procédé selon l'une quelconque des revendications précédentes, dans lequel la signature de pression présente une forme d'onde ayant une amplitude inférieure à 10 % de la valeur de la pression d'occlusion, de préférence encore inférieure à 5 % de ladite valeur.

7. Procédé selon l'une quelconque des revendications précédentes, dans lequel la signature de pression présente une forme d'onde comprenant une pluralité d'impulsions de pression, de préférence dans lequel la forme d'onde comprend une pluralité de groupes d'impulsions de pression, chaque groupe comprenant une pluralité d'impulsions de pression spécifiques.

8. Procédé selon l'une quelconque des revendications précédentes, dans lequel une pluralité de capteurs de suivi d'écoulement du sang sont disposés sur le sujet, de préférence dans lequel le procédé comprend en outre la sélection d'un ou de plusieurs capteurs au sein de la pluralité de capteurs pour optimiser la mesure de la pression artérielle.

9. Procédé selon l'une quelconque des revendications précédentes, dans lequel, dans le cas où une mesure de la pression artérielle du sujet ne peut pas être fournie, une indication correspondante est fournie à l'utilisateur.

10. Appareil (2) pour mesurer la pression artérielle, l'appareil comprenant :

un moyen (6) pour appliquer une pression d'occlusion à une partie du corps (4) d'un sujet, de façon à ce que l'écoulement du sang à travers un vaisseau sanguin puisse être bloqué ;
un moyen (14) pour augmenter la pression d'occlusion ; et
un capteur (20) pour détecter des modifications concernant l'écoulement du sang à travers le vaisseau sanguin suite à la pression appliquée ; caractérisé comme comprenant en outre :

un moyen (16) pour appliquer une signature de pression superposée sur la pression d'occlusion à la partie du corps (4) du sujet ;
un moyen (22) pour déterminer quand les modifications concernant l'écoulement du sang correspondent aux caractéristiques de la signature de pression ; et
un moyen pour déterminer la pression artérielle sur la base des modifications concernant l'écoulement du sang.

11. Appareil (2) selon la revendication 10, dans lequel le moyen (6) pour appliquer la pression d'occlusion au sujet comprend un brassard qui va être enroulé autour d'un membre (4) du sujet, de préférence dans lequel le brassard comprend une première partie de brassard (8) pour appliquer la pression d'occlusion au sujet et une seconde partie de brassard (10) à l'intérieur de laquelle se trouvent un ou plusieurs capteurs d'écoulement du sang (20).

12. Appareil (2) selon l'une ou l'autre des revendications 10 ou 11, dans lequel le moyen (14) pour appliquer la pression d'occlusion et le moyen (16) pour appliquer la signature de pression sont les mêmes.

13. Appareil (2) selon l'une quelconque des revendications 10 à 12, dans lequel le capteur (20) pour détecter les modifications concernant l'écoulement du sang à travers le vaisseau sanguin réagit à un écoulement du sang nul.

14. Appareil (2) selon l'une quelconque des revendications 10 à 13, dans lequel le capteur est un capteur Doppler (20).

15. Appareil selon l'une quelconque des revendications 10 à 14, comprenant une pluralité de capteurs disposés sous la forme d'un réseau (20), de manière à être disposés autour du vaisseau sanguin du sujet quand l'appareil (2) est utilisé, de préférence comprenant en outre un moyen pour sélectionner les données de sortie en provenance d'un ou de plusieurs de la pluralité de capteurs du réseau (20).

Figure 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4718426 A
- US 20020147402 A
- GB 2381076 A
- WO 2007015153 A
- WO 2007092680 A
- JP 2003135412 B
- WO 2007017661 A
- WO 2007069155 A
- WO 2008015921 A